# EUROPÄISCHE PATENTSCHRIFT

(11) **EP 2 276 432 B1**
(45) Veröffentlichungstag und Bekanntmachung des Hinweises auf die Patenterteilung: **25.01.2012**
(21) Anmeldenummer: 09749497.5
(22) Anmeldetag: 20.05.2009
(51) Int. Cl.: A61F 5/01

(54) **VERBINDUNGSELEMENT FÜR ORTHOPÄDISCHE KOMPONENTEN**
CONNECTING ELEMENT FOR ORTHOPEDIC COMPONENTS
ÉLÉMENT DE RACCORDEMENT POUR COMPOSANTS ORTHOPÉDIQUES

(30) Priorität: 20.05.2008 DE 102008024750
(43) Veröffentlichungstag der Anmeldung: 26.01.2011
(73) Patentinhaber: Otto Bock HealthCare GmbH, 37115 Duderstadt (DE)
(72) Erfinder: AUBERGER, Roland, A-1020 Wien (AT); FRIESEN, Jeff, UT 84120 (US)
(74) Vertreter: Stornebel, Kai
(86) Internationale Anmeldenummer: PCT/DE2009/000733
(87) Internationale Veröffentlichungsnummer: WO 2009/140955

(56) Entgegenhaltungen:
- WO-A-2006/053283
- US-A- 5 088 479
- US-A1- 2007 027 421

## Beschreibung

Die Erfindung betrifft Verbindungselement zwischen zwei starr miteinander gekoppelten orthopädischen Komponenten, insbesondere Orthesen- oder Prothesenkomponenten, mit einem Oberteil und einem Unterteil. Eine starre Verbindung liegt dann vor, wenn keine Verschwenkung um eine festgelegte Schwenkachse möglich ist. Die starre Verbindung ist auch dann gegeben, wenn das Verbindungselement nachgiebig in einer Aufnahme gelagert ist. Eine elastische Ausgestaltung des Verbindungselementes, insbesondere eine Biegeelastizität steht einer starren Verbindung nicht entgegen. Ein solches Verbindungselement ist insbesondere zum Einsatz in einer sogenannten Knie-Knöchel-Fuß-Orthese geeignet und dient zur Verbindung eines Fußteiles mit einer Unterschenkelstruktur, die fest an dem Unterschenkel eines Orthesenträgers angeordnet wird. Eine solche Orthese dient zur Unterstützung des Bewegungsapparates. Die Erfindung betrifft auch eine Ortheseneinrichtung mit einem solchen Verbindungselement.

Aus der US 2008/0039756 A1 ist eine Orthese mit einem Rahmen bekannt, dessen proximaler Rahmenteil an dem Oberschenkel eines Orthesenträgers festgelegt werden kann. Ein distales Rahmenteil ist über ein Kniegelenk an dem proximalen Rahmenteil verschwenkbar gelagert. An dem distalen Ende des distalen Rahmenteils ist ein Fußteil gelenkig befestigt. Über einen Aktuator kann das Fußteil angetrieben verschwenkt werden. Das proximale Rahmenteil kann gegenüber dem distalen Rahmenteil über ein Knieaktuator verlagert werden. Eine solche Orthese dient zur aktiven Unterstützung des Bewegungsablaufes.

Die WO 2005/058211 A2 beschreibt eine gattungsgemäße Orthese mit medial und lateral angeordneten Streben, die jeweils über ein Kniegelenk miteinander verbunden sind. Die Streben werden über Bügel, an denen die medialen und lateralen Streben befestigt sind, in einem definierten Abstand zueinander gehalten. An dem distalen Ende des Oberschenkelteils und dem proximalen Ende des Unterteils sind die Bügel so ausgestaltet, dass sie eine Aufnahme für einen Hydraulikzylinder bilden. Der Hydraulikzylinder ist dorsal angeordnet, wenn die Orthese getragen wird. Nachteilig hieran ist, dass die Dämpfereinrichtung sehr viel Platz benötigt und in der Regel nicht unter einer normalen Hose getragen werden kann.

Die Verwendung einer Balkenfeder aus einem faserverstärkten Verbundwerkstoff ist beispielsweise im Zusammenhang mit einer Fußheberorthese aus der DE 10 2004 027 252.2 bekannt. An der gebogen ausgebildeten Balkenfeder sind Befestigungseinrichtungen zur Festlegung der Feder an dem Fuß und dem Unterschenkel angeordnet.

Die US 5,088,479 beschreibt eine Knöchel-Fuß-Orthese mit einem Wadenpolster und einer Fußauflage, die über ein Verbindungselement in Gestalt einer Blattfeder aus einer Metalllegierung oder einem Carbonfaserverbundwerkstoff miteinander verbunden sind. Über Befestigungselemente kann die Orthese an dem Fuß und Unterschenkel festgelegt werden.

Die US 2007/0027421 A1 beschreibt ein dynamische Orthesensteuerungssystem für eine Fuß-Knöchel-Orthese mit einem Fußteil, einer Wadenabstützung und einer Schienbeinabstützung, wobei die Waden- und Schienbeinabstützung über eine Blattfeder mit dem Fußteil verbunden sind.

Die WO 2006/053283 A2 beschreibt eine Knöchel-Fuß-Orthese mit einem Fußteil und einem daran über langgestreckte Widerstandselemente befestigten Befestigungselement zum Befestigen an der Wade des Nutzers. Ein Sensor zum Ermitteln des Lastfaktors kann vorgesehen sein.

Aufgabe der vorliegenden Erfindung ist es, ein Verbindungselement insbesondere für Ortheseneinrichtungen oder Protheseneinrichtungen bereitzustellen, mit dem eine verbesserte Erfassung von wirksamen Kräften zwischen den verbundenen Komponenten erreicht wird. Erfindungsgemäß wird diese Aufgabe durch ein Verbindungselement mit den Merkmalen des Hauptanspruches gelöst. Vorteilhafte Ausgestaltungen und Weiterbildungen der Erfindung sind in den Unteransprüchen aufgeführt.

Das erfindungsgemäße Verbindungselement zwischen zwei starr miteinander gekoppelten orthopädischen Komponenten mit einem Oberteil und einem Unterteil sieht vor, dass das Verbindungselement einen Laminataufbau aufweist und in dem Verbindungselement zumindest ein Sensor zur Ermittlung eines wirksamen Momentes oder wirksamer Kräfte angeordnet ist. Über den Sensor oder die Sensoren kann das jeweils momentan wirksame Moment, z.B. Torsions- oder Biegemoment ermittelt werden, woraus Rückschlüsse über die momentane Strukturbelastung oder beispielsweise die momentane Gangphase gezogen werden können. Eine starre Verbindung liegt vor, wenn das Oberteil und das Unterteil nicht gelenkig miteinander verbunden sind.

Die Sensorik zur Ermittlung der Kräfte oder Momente ist bevorzugt innerhalb des Verbindungselementes einlaminiert, also von den Laminatschichten umgeben, so dass der Sensor gegenüber äußeren Einflüssen weitestgehend abgeschirmt ist und nur die wirksamen Kräfte und Momente innerhalb des Verbindungselementes erfasst. Gleiches glt für eine einlaminierte Signalaufbereitung oder Verstärkerschaltung, die sich bevorzugt in der Nähe der Sensorik befindet

Um Störungen aufgrund des einlaminierten Zustandes zu minimieren, sieht eine Weiterbildung der Erfindung vor, dass jeder Sensor gegenüber unerwünschten Scherkräften des Laminatwerkstoffes abgeschirmt ist, bevorzugt erfolgt dies dadurch, dass zwischen dem Sensor und dem Laminatwerkstoff eine Trennschicht, insbesondere eine Folie oder ein

Schrumpfschlauch angeordnet ist. Dadurch wird vermieden, dass Scherkräfte innerhalb des Laminatwerkstoffes bei einer Biegung des Verbindungselements auf den Sensor übertragen werden, wodurch die Messung verfälscht werden würde. Die Trennschicht kann einseitig an dem Sensor angeordnet sein und nur eine Seite des Sensors gegenüber Scherkräften abschirmen..

Der Sensor ist bevorzugt als eine Dehnmessstreifenanordnung ausgebildet, die auf einem Träger angeordnet ist, wobei auch der Träger bevorzugt in dem Verbindungselement einlaminiert ist. Das Einlaminieren des Trägers erfolgt dabei insbesondere dergestalt, dass der Träger die Verformungen des Verbindungselementes mitmacht, so dass aus der Verformung des Trägers auf die Belastung des Verbindungselementes geschlossen werden kann. Dadurch wird ein Modul bereitgestellt, das aus dem Träger und einem vorher darauf aufgebrachten Dehnmessstreifen, ggf. gemeinsam mit einer Schaltung zur Auswertung, Aufbereitung und Verstärkung des Sensorsignals besteht. Dieses Modul kann einfach in das Verbindungselement bei dessen Herstellung einlaminiert werden, wobei Anschlüsse zur Übermittlung der Sensordaten aus dem Verbindungselement heraus geführt werden müssen, beispielsweise in Gestalt von Kabeln oder einlaminierten Steckerbuchsen. Die Übermittlung der Sensordaten kann auch kabellos, z.B. über eine induktive Koppelung oder Funk erfolgen. Für die Funkübertragung ist insbesondere die Verwendung von auf SAW-Technologie (surface acoustic wave) basierenden Dehnmessstreifen vorteilhaft. Diese können kabellos abgefragt werden, ihr Messprinzip basiert auf der Tatsache, dass die Ausbreitungsgeschwindigkeit einer akustischen Oberflächenwelle in einem Kristall und daher die Signallaufzeit abhängig von dessen mechanischen Spannungszustand ist.

Die gesamte Sensoreinheit kann auf einer einzigen Platine ausgeführt sein, auf der sich auch die Auswerteelektronik befindet. Dabei ist vorgesehen, dass der Sensor, beispielsweise ein Dehnmessstreifen, auf der Platine mittels Dickschichttechnologie aufgebracht ist. Es wird dadurch kein separates Trägermaterial benötigt, so dass insgesamt ein kompakter Aufbau verwirklicht werden kann. Die Platine dient somit als Träger des Sensors und der integrierte Schaltkreise der Auswerteelektronik. Die Daten des Sensors werden ggf. nach der Auswertung durch die Auswerteelektronik, an eine Steuereinrichtung übermittelt, die mit dem Sensor verbunden ist. Die Steuereinrichtung kann ebenfalls auf der Platine angeordnet sein. Die Steuereinrichtung ist mit einem Aktuator gekoppelt, so dass auf der Grundlage der Sensordaten das Verhalten des Aktuators verändert, also variiert werden kann.

Die Trennschicht kann an dem Träger befestigt sein, so dass ein eingekapselter Sensor in den Laminatwerkstoff eingearbeitet werden kann, ohne dass es weiterer Arbeitsschritte bedarf. Die Trennschicht, beispielsweise als Folie ausgebildet, kann auf dem Träger aufgeklebt werden, so dass lediglich die auf den Träger übertragenden Biegekräfte oder Biegemomente von dem Sensor gemessen werden. Statt einer Folie kann auch ein Schrumpfschlauch eingesetzt werden. Eine Weiterbildung der Erfindung sieht vor, dass der Sensor und ggf. der Träger schwimmend in dem Verbindungselement gelagert sind, um zu vermeiden, dass bei einer z.B. bei einer thermisch bedingten Ausdehnung des Trägers während der Benutzung der Träger in sich verformt wird, wenn der Laminatwerkstoff sich nicht im gleichen Maß ausdehnt. Dies wäre beispielsweise bei einer Werkstoffpaarung von faserverstärkten Verbundwerkstoffen und einem Metallträger der Fall. Eine schwimmende Lagerung vermeidet einerseits die Erzeugung eines fehlerhaften Signals und andererseits eine Sprengung des einkapselnden Werkstoffes des Verbindungselementes.

Der Träger und damit auch der Dehnmessstreifen sind bevorzugt in der neutralen Faser des Verbindungselementes angeordnet, wodurch eine störgrößenfreie Messung der Biegung des Verbindungselementes gewährleistet ist. Träger unterschiedlicher Dicke können einlaminiert werden, um die Empfindlichkeit des Sensoren variieren zu können. Je dicker der Träger ist, desto empfindlicher ist der Sensor, da die Verzerrungen an dem Dehnmessstreifen bei gegebener Verformung höher sind.

Der Träger kann als eine Platte, z.B. eine Kunststoff- oder Metallplatte oder als stabförmiger, insbesondere zylindrischer oder hülsenförmiger Träger ausgebildet sein. Es können mehrere Sensoren oder Dehnmessstreifen auf einem Träger angeordnet sein, insbesondere können Dehnmessstreifen an gegenüberliegenden Seiten des Trägers angeordnet sein, um Biege- und Torsionsmomente störungsfrei erfassen zu können. Ebenfalls können mehrere Signalaufbereitungseinrichtungen an einem Träger angeordnet sein. Die Sensoren können entlang der Längserstreckung des Träger beabstandet zueinander an dem Träger und an oder in dem Verbindungselement angeordnet sein.

Eine Ausführungsform der Erfindung sieht vor, dass das Verbindungselement als eine Fersenfeder ausgebildet ist, die lateral oder dorsal zu dem Orthesenträger angeordnet ist. Insbesondere bei einer dorsal zu dem Orthesenträger angeordneten Fersenfeder ist es vorgesehen, dass der Sensor posterior zu dem Knöchel angeordnet ist, wobei die sich aus dem Abstand zu dem eigentlichen Knöchelgelenk ergebende Fehlmessung, der Offset, herausgerechnet werden kann, so dass bei einer bekannten Einbaulage eine Abschätzung des Knöchelmomentes erfolgen kann.

Das Verbindungselement besteht bevorzugt aus einem faserverstärkten Verbundwerkstoff, beispielsweise einem glasfaserverstärkten oder kohlefaserverstärkten Verbundwerkstoff und ist beispielsweise als ein Balkenfederelement ausgebildet.

Das Verbindungselement kann auswechselbar mit dem Oberteil und/oder mit dem Unterteil verbunden sein, um eine leichte Anpassung an unterschiedliche physiologische Gegebenheiten der jeweiligen Träger der orthopädischen Komponenten zu gewährleisten, beispielsweise um eine Anpassung an das Körpergewicht, die physische Leistungsfähigkeit oder die Körpergröße zu ermöglichen. Alternativ dazu ist das Verbindungselement als ein Teil des Oberteils und/oder des Unterteils ausgebildet, so dass beispielsweise ein Fußteil und eine Dorsalfeder einstückig miteinander ausgebildet sind, um mit dem Oberteil in Gestalt einer Unterschenkelstruktur verbunden zu werden. Umgekehrt kann das Verbindungselement mit dem Oberteil einstückig ausgebildet werden, so dass lediglich das Unterteil an dem Verbindungselement angeordnet werden muß. Oberteil und Unterteil haben unterschiedliche Strukturen und/oder Funktionen, beispielsweise Kniegelenk und Fuß oder Vorderfuß und Knöchel, die miteinander gekoppelt werden müssen. Diese Koppelung erfolgt über das Verbindungselement.

Der Sensor kann zu Aufnahme von Axialkräften ausgebildet sein, um eine Druck- oder Zugbelastung innerhalb des Verbindungselementes detektieren zu können. Sofern der Sensor auf der Dehnmessstreifentechnologie basiert, kann ein oder können mehrere Dehnmessstreifen in einer Orientierung schräg zu der Längserstreckung des Trägerelementes appliziert sein. Abhängig von der gewünschten Messrichtung des Sensorelements kann der Sensor auch schräg zur,Längserstreckung der Verbindungselements einlaminiert sein.

Bei einer Ausgestaltung des Verbindungselementes als Verbindungsteil zwischen einem Unterteil eines Kniegelenkes und einem Fußteil und einer dementsprechenden Anordnung des Verbindungselementes im Unterschenkelbereich ist bevorzugt vorgesehen, dass der Sensor oder die Sensoren im Bereich des Knöchelgelenks angeordnet ist oder sind, um das wirksame Knöchelmoment möglicht genau bestimmen zu können.

Mehrere Sensoren können axial zueinander beabstandet an dem Verbindungselement angeordnet sein, um die Genauigkeit der Erfassung der wirksamen Kräfte und Momente zu erhöhen. Neben dem einlaminierten Sensor oder den einlaminierten Sensoren kann zumindest ein weiterer Sensor an der Oberfläche des Verbindungselementes befestigt sein, der ergänzende Daten liefert.

Die Sensordaten können neben einer Signalübertragung via Kabel auch kabellos übertragen werden, um eine entsprechende Steuerung mit den benötigten Signalen zu versorgen.

Die Erfindung betrifft auch eine Ortheseneinrichtung mit einem Oberteil, das eine Befestigungseinrichtung zur Festlegung an einem Oberschenkel aufweist, einem Unterteil, das eine Befestigungseinrichtung zur Festlegung an einem Unterschenkel aufweist, und einem mit dem Unterteil verbundenes Fußteil zur Abstützung eines Fußes, wobei das Oberteil und das Unterteil um eine Gelenkachse schwenkbar miteinander verbunden sind und zwischen dem Oberteil und dem Unterteil ein Aktuator angeordnet ist, mit einem Verbindungselement, wie es oben beschrieben wurde, das zwischen dem Fußteil und dem Unterteil angeordnet ist und diese miteinander verbindet.

Nachfolgend werden Ausführungsbeispiele der Erfindung anhand der beigefügten Figuren näher erläutert. Es zeigen:
- Figur 1-: eine Knie-Knöchel-Fuß-Orthese in Seitenansicht;
- Figur 2-: einen Sensor auf einer Trägerplatte;
- Figur 3-: ein Anordnungsbeispiel von Sensoren gemäß Figur 2 in einem Verbindungselement;
- Figur 4-: eine Variante des Sensors gemäß Figur 2; sowie
- Figur 5-: ein Anordnungsbeispiel des Sensors gemäß Figur 4.

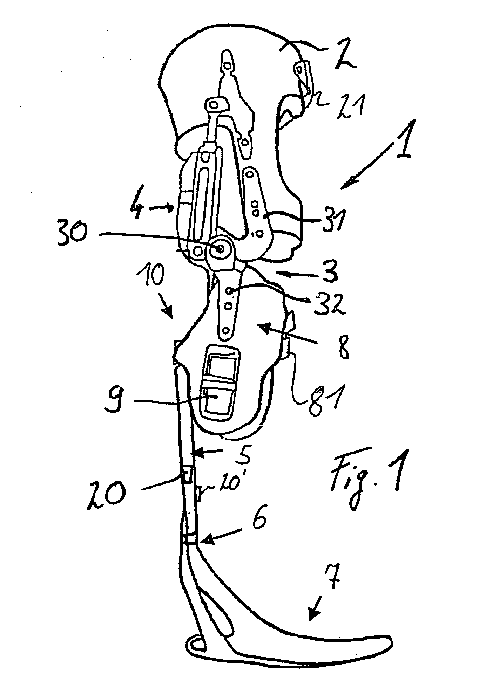

In der Figur 1 ist eine Knie-Knöchel-Fuß-Orthese 1 in Seitenansicht dargestellt. Die Orthese 1 weist eine Oberschenkelstruktur 2 auf, an der eine Gelenkeinrichtung 3 zur gelenkigen Befestigung einer Unterschenkelstruktur 8 vorgesehen ist. Die Befestigung der Gelenkeinrichtung 3 erfolgt über Gelenkstrukturelemente 31, 32. Über einen Aktuator 4, der im vorliegend Ausführungsbeispiel als eine Hydraulikeinrichtung ausgebildet ist, die gegebenenfalls mit einem weiteren Aktuator gekoppelt sein kann, kann die Bewegung zwischen der Oberschenkelstruktur 2 und der Unterschenkelstruktur 8 kontrolliert werden, beispielsweise indem unterschiedliche Dämpfungsgrade der Bewegung zwischen der Oberschenkelstruktur 2 und der Unterschenkelstruktur 8 eingestellt werden. Die Unterschenkestruktur 8 verschwenkt um die Gelenkachse 30 relativ zu der Oberschenkelstruktur 2, wobei die Oberschenkel- und Unterschenkelstruktur 2, 8 über Befestigungseinrichtungen 21, 81 an dem Ober- und Unterschenkel des Orthesenträgers festgelegt sind.

An der Unterschenkelstruktur 8 ist ein Fußteil 7 über ein Verbindungselement 5 verbunden, das in dem vorliegenden Ausführungsbeispiel als Balkenfederelement ausgebildet ist. Das Fußteil 7 ist an dem Verbindungselement 5 über einen Adapter 6 befestigt. Das Fußteil 7 und der Adapter 6 können entweder einteilig ausgebildet oder aus mehreren Komponenten zusammengesetzt werden. Auch an der Unterschenkelstruktur 8 ist ein Adapter 10 vorgesehen, mit dem es möglich ist, das Balkenfederelement 5 auswechselbar an der Orthese 1 zu befestigen, wobei die Unterschenkelstruktur 8 als Oberteil und das Fußteil 7 als Unterteil angesehen wird. Zur Befestigung werden die Enden des Verbindungselementes 5 in den jeweiligen Adapter 6, 10 eingeschoben und dort fixiert, beispielsweise über Schrauben. Das Ausbilden von Klemmadaptern 6, 10 ist zu bevorzugen, da das Bohren von Löchern in Faserverbundwerstoffen einerseits aufwendig ist und andererseits zu einer erheblichen Schwächung der Festigkeit des Faserverbundwerkstoffes des Verbindungselementes 5 führt. Alternativ zu einer Ausgestaltung der Verbindung zwischen dem Verbindungselement 5 und dem Fußteil 7 und der Unterschenkelstruktur 8 über Adapter 6, 10 kann das Verbindungselement 5 auch einstückig mit dem Fußteil 7 und/oder der Unterschenkelstruktur 8 ausgebildet sein.

Im dem dargestellten Ausführungsbeispiel ist das Verbindungselement 5 dorsal angebracht, grundsätzlich ist es auch möglich, ein solches Verbindungselement 5 als Feder auch frontal, medial und/oder lateral vorzusehen, wobei ein Austausch der Federn weiterhin möglich bleibt. Die dorsale Anordnung der Feder 5 hat den Vorteil, dass eine sehr schlanke Silhouette beim Orthesenträger erreicht wird, so dass die Orthese so unauffällig wie möglich getragen werden kann. Weiterhin ermöglicht diese Anordnung die Realisierung einer möglichst langen Balkenfeder, was sich positiv auf die Federeigenschaften und die Haltbarkeit des Federelementes 5 auswirkt.

In dem Verbindungselement 5 ist ein Sensor 20 zur Ermittlung des Biegemomentes oder anderer wirksamer Kräfte innerhalb des Verbindungselementes 5 angeordnet. Die Sensordaten können einer Steuerungseinrichtung 9 übermittelt werden, die das Verhalten des Aktuators 4 variieren kann. Der Aktuator 4 kann als aktives Stellelement ausgebildet sein, ebenfalls ist eine Ausgestaltung als rein passiver Dämpfer oder eine Kombination davon möglich. Ein zusätzlicher Sensor 20' ist außen an dem Verbindungselement 5 angeordnet und stellt zusätzliche Daten über die in dem Verbindungselement 5 wirksamen Kräfte und Momente zur Verfügung.

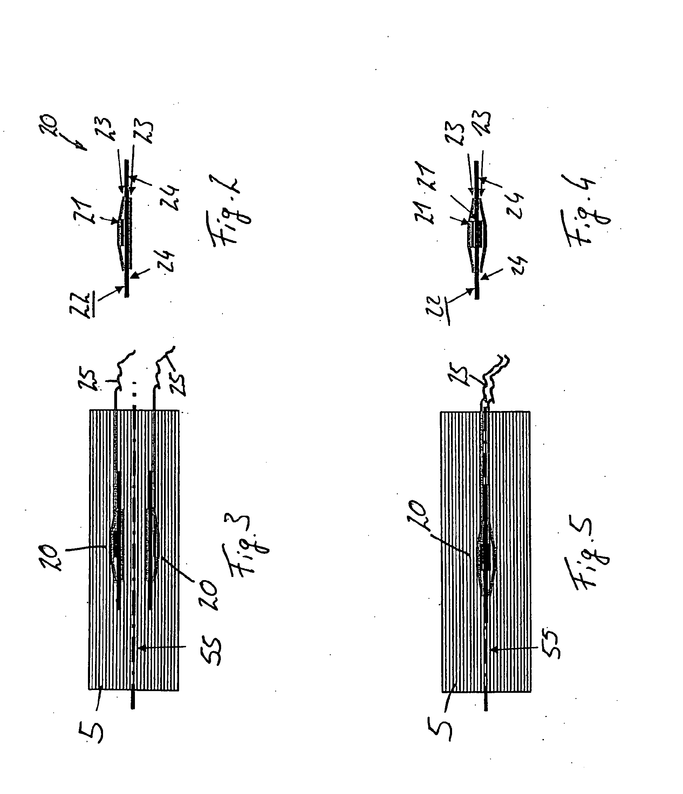

In der Figur 2 ist in einer Querschnittsansicht schematisch die Ausgestaltung eines Sensors 20 mit einem Dehnmessstreifenelement 21 gezeigt, das auf einem Träger 22 befestigt ist. Das Dehnmessstreifenelement 21 ist in herkömmlicher Weise auf dem Träger 22 befestigt, beispielsweise verklebt, und gegenüber der Umgebung durch Trennschichten 23 abgeschirmt. Die Trennschichten 23 sind an dem Träger 22 festgelegt und können beispielsweise aus einer Trennfolie, einem Schrumpfschlauch oder einer Silikoneinbettung oder -beschichtung bestehen. Diese Trennschichten 23, die sowohl auf der Oberseite als auch der Unterseite des Trägers 22 angeordnet sind, verhindern, dass Scherkräfte auf den Träger 22 und den darauf befestigten Dehnmessstreifen 21 übertragen werden. Seitlich neben den Trennschichten 23 sind Verbindungsbereiche 24 des Trägers 22 vorgesehen, an denen der Träger 22 mit dem umgebenen Verbundmaterial verbunden werden kann. Diese Verbindungsbereiche 24 treten in Verbindung mit den Bindemitteln des Faserverbundwerkstoffes und stellen so eine Verbindung zu den Faserwerkstoffen her. In bestimmen Fällen ist es vorteilhaft, wenn der Träger 22 nicht mit dem Faserverbundwerkstoff verbunden ist, da dadurch eine bessere Temperaturkompensation erreicht werden kann. In diesem Fall ist der gesamte Sensor 20 mit einer Trennschicht 23 zu überziehen, so dass sich eine schwimmende Lagerung innerhalb des Faserverbundwerkstoffes ergibt.

In der Figur 3 ist in einer schematischen Seitenansicht das Verbindungselement 5 beispielsweise in Gestalt eines Balkenfederelementes dargestellt. Beidseitig der neutralen Faser 55 ist je ein Sensor 20 mit einem abgeschirmten Dehnmessstreifen 21 auf einem Träger 22 einlaminiert. Kabel 25, die mit dem Dehnmessstreifen 21 verbunden sind, führen aus dem Verbindungselement 5 heraus und zu einer Verstärker- und Signalaufbereitungselektronik, die ebenfalls einlaminiert sein kann, und gegebenenfalls einer Steuereinrichtung. Der Träger 22 ist bevorzugt dünn ausgebildet, beispielsweise zwischen 0,1 mm und 0,5 mm, sodass die gesamte Anordnung des Sensors 20 mit den Dehnmessstreifen 21 und Trennschichten 23 eine Dicke von unter 1 mm aufweist. Die Empfindlichkeit der Sensoren kann durch die Lage innerhalb des Verbindungselementes 5 eingestellt werden, wobei die Veränderung sich durch die Variation der Entfernung zu der neutralen Faser 55 ergibt.

Eine Variante der Erfindung ist in den Figuren 4 und 5 dargestellt, bei der beidseitig auf dem Träger 22 Dehnmessstreifen 21 angeordnet und von Trennschichten 23 umgeben sind. Auch hier sind neben den Trennschichten 23 Verbindungsbereiche 24 vorgesehen, um in das Verbindungselement 5 einlaminiert zu werden. Die Verbindungsbereiche 24 sind jedoch nicht zwingend erforderlich. In der Figur 5 ist in Schnittdarstellung die Anordnung des Sensors 20 gemäß Figur 4 innerhalb des Verbindungselementes 5 dargestellt. Die Anordnung des Sensors 20 erfolgt in der neutralen Faser 55 des Verbindungselementes 5, die Kabel 25 führen aus dem Verbindungselement 5 heraus. Der Träger 22 biegt sich zusammen mit dem Verbindungselement 5 und erzeugt ein Ausgangssignal. Aufgrund der unmittelbar zu der neutralen Faser 55 benachbarten Anordnung der Dehnmessstreifen 21 wird ein verhältnismäßig hoher Verstärkungsfaktor benötigt, um die notwendige Empfindlichkeit des Sensors zu erreichen. Die Empfindlichkeit kann auch über die Dicke des Trägers 22 variiert werden. Beim Design der Sensoreinheit ist ein Kompromiss zwischen Empfindlichkeit, Dicke des Sensors und Verstärkungsfaktor zu finden. Jedoch ergibt sich der Vorteil, dass nur ein einziger Fremdkörper innerhalb des Verbundwerkstoffelementes 5 angeordnet werden muss. Dieser Fremdkörper in Gestalt des Sensors 20, ggf. zusammen mit der Verstärker- und Auswerteelektronik, kann als vollständig vorgefertigtes Modul einfach eingelegt und einlaminiert werden.

## Patentansprüche

1. Verbindungselement zwischen zwei starr miteinander gekoppelten orthopädischen Komponenten, insbesondere Othesenkomponenten, mit einem Oberteil und einem Unterteil wobei das Verbindungselement (5) einen Laminataufbau aufweist, **dadurch gekennzeichnet, dass** in dem Verbindungselement (5) zumindest ein Sensor (20) zur Ermittlung eines Momentes oder einer Kraft angeordnet ist.

2. Verbindungselement nach Anspruch 1, **dadurch gekennzeichnet, dass** der Sensor (20) innerhalb des Verbindungselementes (5) einlaminiert ist.

3. Verbindungselement nach Anspruch 1 oder 2, **dadurch gekennzeichnet, dass** der Sensor (20) gegenüber Scherkräften des Laminatwerkstoffes abgeschirmt ist.

4. Verbindungselement nach einem der voranstehenden Ansprüche, **dadurch gekennzeichnet, dass** zwischen dem Sensor (20) und dem Laminatwerkstoff eine Trennschicht (23), insbesondere eine Folie, angeordnet ist.

5. Verbindungselement nach einem der voranstehenden Ansprüche, **dadurch gekennzeichnet, dass** der Sensor (20) als Dehnmessstreifen (21) ausgebildet ist, der auf einem Träger (22) angeordnet ist.

6. Verbindungselement nach Anspruch 5, **dadurch gekennzeichnet, dass** der Dehnmessstreifen (21) auf einer Platine, die als Träger(22) dient, einer Auswerteelektronik angeordnet ist.

7. Verbindungselement nach einem der Ansprüche 5 bis 6, **dadurch gekennzeichnet, dass** die Trennschicht (23) an dem Träger (22) befestigt ist.

8. Verbindungselement nach einem der Ansprüche 5 bis 7, **dadurch gekennzeichnet, dass** der Träger (22) in der neutralen Faser (55) des Verbindungselementes (5) angeordnet ist.

9. Verbindungselement nach einem der Ansprüche 5 bis 8 **dadurch gekennzeichnet, dass** der Träger (22) schwimmend in dem Verbindungselement (5) gelagert ist.

10. Verbindungselement nach einem der Ansprüche 5 bis 9, **dadurch gekennzeichnet, dass** Träger (22) unterschiedlicher Dicke eingesetzt werden, die in dem Verbindungselement (5) einlaminiert sind.

11. Verbindungselement nach einem der voranstehenden Ansprüche, **dadurch gekennzeichnet, dass** das Verbindungselement (5) als eine Fersenfeder ausgebildet ist, die lateral oder dorsal zu einem Orthesenträger angeordnet ist.

12. Verbindungselement nach einem der voranstehenden Ansprüche, **dadurch gekennzeichnet, dass** der Sensor (20) als Biegemoment- oder Torsionsmomentsensor ausgebildet und/oder Axialkräfte erfassen kann.

13. Verbindungselement nach einem der voranstehenden Ansprüche, **dadurch gekennzeichnet, dass** der Sensor (20) als Dehnmessstreifen ausgebildet und schräg zu der Längserstreckung des Verbindungselements (5) orientiert einlaminiert ist.

14. Verbindungselement nach einem der voranstehenden Ansprüche, **dadurch gekennzeichnet, dass** das Verbindungselement (5) im Unterschenkelbereich angeordnet und der Sensor (20) oder die Sensoren (20) im Bereich des Knöchelgelenks angeordnet ist oder sind.

15. Verbindungselement nach einem der voranstehenden Ansprüche, **dadurch gekennzeichnet, dass** eine Signalverarbeitungsschaltung zur Verstärkung und Aufbereitung der Sensorsignale in dem Verbindungselement (5) einlaminiert ist.

16. Verbindungselement nach einem der voranstehenden Ansprüche, **dadurch gekennzeichnet, dass** zumindest ein weiterer Sensor (20') an der Oberfläche des Verbindungselements (5) angeordnet ist.

17. Ortheseneinrichtung mit einem Oberteil (2), das eine Befestigungseinrichtung (21) zur Festlegung an einem Oberschenkel aufweist, einem Unterteil (8), das eine Befestigungseinrichtung (81) zur Festlegung an einem Unterschenkel aufweist, und einem mit dem Unterteil (8) verbundenes Fußteil (7) zur Abstützung eines Fußes, wobei das Oberteil (2) und das Unterteil (8) um eine Gelenkachse (30) schwenkbar miteinander verbunden sind und zwischen dem Oberteil (2) und dem Unterteil (8) ein Aktuator (4) angeordnet ist, mit einem Verbindungselement (5) nach einem der voranstehenden Ansprüche, das zwischen dem Fußteil (7) und dem Unterteil (8) angeordnet ist und diese miteinander verbindet.

## Claims

1. A connecting element between two rigidly coupled orthopedic components, in particular orthosis components, with an upper part and a lower part, the connecting element (5) has a laminate structure, **characterized in that** at least one sensor (20) for determining a moment or a force is arranged in the connecting element.

2. The connecting element as claimed in claim 1, **characterized in that** the sensor (20) is laminated inside the connecting element (5).

3. The connecting element as claimed in claim 1 or 2, **characterized in that** the sensor (20) is screened off from shearing forces of the laminate material.

4. The connecting element as claimed in one of the preceding claims, **characterized in that** a separating layer (23), in particular a film, is arranged between the sensor (20) and the laminate material.

5. The connecting element as claimed in one of the preceding claims, **characterized in that** the sensor (20) is designed as a strain gauge (21), which is arranged on a substrate (22).

6. The connecting element as claimed in claim 5, **characterized in that** the strain gauge (21) is arranged on a circuit board, which serves as substrate (22), of evaluation electronics.

7. The connecting element as claimed in one of claims 5 through 6, **characterized in that** the separating layer (23) is secured on the substrate (22).

8. The connecting element as claimed in one of claims 5 through 7, **characterized in that** the substrate (22) is arranged in the neutral fiber (55) of the connecting element (5).

9. The connecting element as claimed in on of claims 5 through 8, **characterized in that** the substrate (22) is mounted floating in the connecting element (5).

10. The connecting element as claimed in on of claims 5 through 9, **characterized in that** substrates (22) of different thickness are used, which are laminated in the connecting element (5).

11. The connecting element as claimed in one of the preceding claims, **characterized in that** the connecting element (5) is designed as a heel spring, which is arranged laterally or dorsally with respect to an orthosis wearer.

12. The connecting element as claimed in one of the preceding claims, **characterized in that** the sensor (20) is designed as a bending moment or torsional moment sensor and/or can detect axial forces.

13. The connecting element as claimed in one of the preceding claims, **characterized in that** the sensor (20) is designed as a strain gauge and is laminated in an oblique orientation with respect to the longitudinal extent of the connecting element (5).

14. The connecting element as claimed in one of the preceding claims, **characterized in that** the connecting element (5) is arranged in the lower leg area, and the sensor (20) or the sensors (20) is or are arranged in the area of the ankle joint.

15. The connecting element as claimed in one of the preceding claims, **characterized in that** a signal processing circuit for amplifying and conditioning of the sensor signals is laminated in the connecting element (5).

16. The connecting element as claimed in one of the preceding claims, **characterized in that** at least one further sensor (20') is arranged on the surface of the connecting element (5).

17. An orthosis device with an upper part (2), which has a fastening means (21) for securing to a thigh, a lower part (8), which has a fastening means (81) for securing to a lower leg, and a foot part (7) connected to the lower part (8) and supporting a foot, the upper part (2) and the lower part (8) being connected pivotably to each other about a joint axis (30), and an actuator (4) being arranged between the upper part (2) and the lower part (8), and with a connecting element (5) which is claimed in one of the preceding claims and which is arranged between the foot part (7) and the lower part (8) and connects these to each other.

## Revendications

1. Elément de raccordement entre deux composants orthopédiques couplés rigidement l'un avec l'autre, en particulier des composants d'orthèse, comprenant une partie supérieure et une partie inférieure, l'élément de raccordement (5) présentant une structure stratifiée, **caractérisé en ce qu'**au moins un capteur (20) est agencé dans l'élément de raccordement (5) pour déterminer un moment ou une force.

2. Elément de raccordement selon la revendication 1, **caractérisé en ce que** le capteur (20) est stratifié à l'intérieur de l'élément de raccordement (5).

3. Elément de raccordement selon la revendication 1 ou 2, **caractérisé en ce que** le capteur (20) est protégé des efforts tranchants du matériau stratifié.

4. Elément de raccordement selon l'une quelconque des revendications précédentes, **caractérisé en ce qu'**une couche de séparation (23), en particulier un film, est agencée entre le capteur (20) et le matériau stratifié.

5. Elément de raccordement selon l'une quelconque des revendications précédentes, **caractérisé en ce que** le capteur (20) est formé par une jauge d'élongation (21) qui est agencée sur un support (22).

6. Elément de raccordement selon la revendication 5, **caractérisé en ce que** la jauge d'élongation (21) est agencée sur une platine d'une électronique d'exploitation, la platine servant de support (22).

7. Elément de raccordement selon l'une quelconque des revendications 5 à 6, **caractérisé en ce que** la couche de séparation (23) est fixée au support (22).

8. Elément de raccordement selon l'une quelconque des revendications 5 à 7, **caractérisé en ce que** le support (22) est agencé dans la fibre neutre (55) de l'élément de raccordement (5).

9. Elément de raccordement selon l'une quelconque des revendications 5 à 8, **caractérisé en ce que** le support (22) est monté flottant dans l'élément de raccordement (5).

10. Elément de raccordement selon l'une quelconque des revendications 5 à 9, **caractérisé en ce que** des supports (22) de différentes épaisseurs sont mis en oeuvre, lesquels sont stratifiés dans l'élément de raccordement (5).

11. Elément de raccordement selon l'une quelconque des revendications précédentes, **caractérisé en ce que** l'élément de raccordement (5) est formé en tant que ressort de talon qui est agencé latéralement ou dorsalement à un porteur d'orthèse.

12. Elément de raccordement selon l'une quelconque des revendications précédentes, **caractérisé en ce que** le capteur (20) est formé par un capteur de moment de flexion ou de moment de torsion et/ou peut détecter des forces axiales.

13. Elément de raccordement selon l'une quelconque des revendications précédentes, **caractérisé en ce que** le capteur (20) est formé par une jauge d'élongation et est stratifié orienté en biais par rapport à l'extension longitudinale de l'élément de raccordement (5).

14. Elément de raccordement selon l'une quelconque des revendications précédentes, **caractérisé en ce que** l'élément de raccordement (5) est agencé dans la région de la jambe inférieure et le capteur (20) ou les capteurs (20) est ou sont agencé(s) dans la région de l'articulation de la cheville.

15. Elément de raccordement selon l'une quelconque des revendications précédentes, **caractérisé en ce qu'**un circuit de traitement de signal est stratifié dans l'élément de raccordement (5) pour amplifier et traiter les signaux de capteur.

16. Elément de raccordement selon l'une quelconque des revendications précédentes, **caractérisé en ce qu'**au moins un autre capteur (20') est agencé à la surface de l'élément de raccordement (5).

17. Dispositif d'orthèse comprenant une partie supérieure (2) présentant un dispositif de fixation (21) pour la fixation à une cuisse, une partie inférieure (8) présentant un dispositif de fixation (81) pour la fixation à une jambe inférieure, et une partie pied (7) liée à la partie inférieure (8) pour supporter un pied, la partie supérieure (2) et la partie inférieure (8) étant reliées ensemble pivotantes autour d'un axe d'articulation (30) et un actionneur (4) étant agencé entre la partie supérieure (2) et la partie inférieure (8), le dispositif comprenant un élément de raccordement (5) selon l'une quelconque des revendications précédentes qui est agencé entre la partie pied (7) et la partie inférieure (8) et qui relie celles-ci ensemble.
